# EUROPEAN PATENT APPLICATION

(11) **EP 1 170 007 A1**
(43) Date of publication of application: **09.01.2002**
(21) Application number: 00902942.2
(22) Date of filing: 14.02.2000
(51) Int. Cl.: A61K 31/122, A61P 43/00, A61P 1/16, A61P 25/28, A61P 3/10, A61P 35/00, A23L 1/30, A23L 2/00

(54) **REMEDIES**

(30) Priority: 19.02.1999 JP 4223699; 15.04.1999 JP 10849999; 17.09.1999 JP 26453999
(71) Applicant: Takara Shuzo Co, Ltd., Kyoto-shi, Kyoto 612-8061 (JP)
(72) Inventor: OHNOGI, Hiromu, c/o Takara Shuzo Co.,Ltd., Kyoto-shi, Kyoto 612-8061 (JP); AKIYAMA, Kaori, c/o Takara Shuzo Co.,Ltd., Kyoto-shi, Kyoto 612-8061 (JP); TOMINAGA, Takanari, Otsu-shi, Shiga 520-0242 (JP); NISHIYAMA, Eiji, Moriyama-shi, Shiga 524-0102 (JP); WU, Hua-Kang, Otsu-shi, Shiga 520-2133 (JP); TATSUMI, Yoko, Kyoto-shi, Kyoto 616-8396 (JP); SAGAWA, Hiroaki, Kusatsu-shi, Shiga 525-0025 (JP); KATO, Ikunoshin, Uji-shi, Kyoto 611-0028 (JP)
(74) Representative: Vossius, Volker, Dr.
(86) International application number: JP0000787
(87) International publication number: WO0048586

(57) **Abstract**

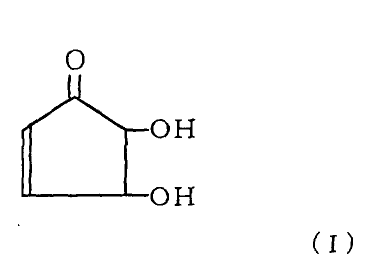

Remedies or preventives for diseases with a need for the reinforcement of the production of growth factor and/or diseases with a need for the reinforcement of the production of interleukin-12, characterized by containing as the active ingredient a compound selected from among 4,5-dihydroxy-2-cyclopenten-1-one represented by formula (I), 4-hydroxy-2-cyclopenten-1-one and derivatives thereof.

## Description

### Technical Field

The present invention relates to a pharmaceutical composition, a formulation, or a food or a drink, which is effective for a disease that requires enhancement of cell growth factor production for its treatment or prevention.

### Background Art

Growth factors are a group of substances that promote growth/proliferation of cells in a trace amount. They control proliferation, elongation, enlargement, differentiation and the like of cells. Known physiological functions of growth factors include an activity of promoting growth, an insulin-like activity, anabolism, an osteogenetic activity, an activity of promoting cell growth, a neurotrophic factor-like activity, an erythropoietin-like activity, an activity of promoting intrauterine growth, an activity of increasing renal bloodstream/protecting renal cells, and an immunopotentiating activity. Thus, they are expected to ameliorate unresponsiveness to growth hormone, diabetes and disassimilation state, and to have applications to diseases such as osteoporosis, diseases that require tissue repair for their treatment or prevention, neurodegenerative diseases, anemia, insufficient intrauterine growth, renal insufficiency and immunodeficiency.

The application of a growth factor to a disease is usually performed by administering the growth factor from the outside. However, it is difficult to obtain a growth factor in large quantities. Furthermore, although a peptidergic growth factor can be prepared by gene engineering, such a growth factor results in low sensitivity in many cases. Then, it is required to administer a large amount of the growth factor. It is known that the administration of a large amount of the growth factor is accompanied by various side effects including pyrexia and anorexia.

Liver rapidly regenerates to the original size after it is subjected to partial hepatectomy. The essential factor involved in the liver regeneration was unknown for a long time. Then, hepatocyte growth factor (HGF) was found in plasma from patients with fulminant hepatitis and isolated and purified therefrom [Gohda, E. et al. J. Clin. Invest., 88:414-419 (1988)]. Furthermore, the cDNA for human HGF was cloned, and the primary structure of HGF was revealed [Miyakawa, K. et al., Biochem. Biophys. Res. Commun., 163:967-973 (1989)]. Furthermore, it was demonstrated that scatter factor (SF), which activates the motility of cells, and tumor cytotoxic factor (TCF) are the same substance with HGF [Weidner, K.M. et al., Proc. Natl. Acad. Sci. USA, 88:7001-7005 (1991); Shima, N. et al., Biochem. Biophy. Res. Commun., 180:1151-1158 (1991)].

HGF promotes the growth of many types of epithelial cells including bile duct epithelial cells, renal tubule epithelial cells and tunica mucosa ventriculi cells in addition to hepatocytes. Furthermore, HGF is a multifunctional active substance which exhibits a wide variety of physiological activities including activation of motility of epithelial cells as well as induction of morphogenesis such as vascularization and lumen formation of epithelial cells. In other words, HGF promotes growth of epithelial cell for repair of damaged organ, activates motility, and induces morphogenesis such as vascularization and the like in various organs.

HGF also exhibits an activity of proliferating hepatocyte, an activity of promoting protein synthesis, an activity of improving cholestasia, an activity of preventing renal damage due to drug and the like. Based on these facts, HGF is expected to serve as therapeutic agents for severe hepatitis, cirrhosis and cholestasia in liver. However, HGF itself has not been practically used as a therapeutic agent. A gene therapy method·in which the gene for HGF is introduced was attempted, but has a long way to go before practical use because of side effects due to the exertion of the activity at unfavorable time and site.

Nerve cells play the principle role in the maintenance of mental activities of humans including intellectual functions, memory, emotion and behavior. It is considered that neurotrophic factors specific to respective types of the nerve cells are required for the differentiation, survival and functional expression of nerve cells on which these mental activities are based, although such consideration is no better than presumption. The only factor whose existence and function have been clarified is nerve growth factor (hereinafter abbreviated as NGF). Since NGF is the neurotrophic factor for large cholinergic nerve cells in the basal part of forebrain, attention has been given to its relationship with dementia of Alzheimer [Pharmacia, 22(2):147-151 (1986); Ronen Seishin Igaku, 3(6):751-758 (1986)].

Dementia of Alzheimer is one disease type of senile dementia, and is a disease which is clinically accompanied by developmental disability, focal symptom, tonic cramp of inferior limb, ictus epilepticus and the like, and whose pathological observations include senile plaque and Alzheimer neurofibrillary tangles. In the recent aging society, dementia of Alzheimer shows a tendency to increase and has become a serious matter of public concern. However, no particular method of amelioration of the disease state or treatment has been found.

Prominent degeneration and considerable decrease in the choline acetyltransferase (CAT) activity in the basal part of forebrain centering on the Meynert basal ganglia are observed in brains of patients with dementia of Alzheimer [Annu. Rev. Neurosci., 3:77 (1980)]. In 1985, a study using rat brains showed that NGF acts as a neurotrophic factor at this site of the brain [EMBO J., 4:1389 (1985)]. Then, attention was given to the relationship between NGF and this disease. Furthermore, significant elimination of cholinergic nerve cells in addition to GABAergic nerve cells in the striata of the brains of patients with Huntington's chorea is observed. Thus, it was found that NGF also acts on endogenous cholinergic nerve cells in the striata [Science, 234:1341 (1986)]. Thus, it has been pointed out that NGF may be involved in this disease. Furthermore, the effects of NGF have been studied using animals (e.g., rats) which may serve as models for various nerve diseases. It has been reported that, if NGF is intracerebrally administered before nerve cells significantly degenerate in rats, the degeneration and the decrease in the CAT activity can be prevented [J. Neurosci., 6:2155 (1986); Brain Res., 293:305 (1985); Science, 235:214 (1986); Proc. Natl. Acad. Sci. USA, 83:9231 (1986)]. It has also been demonstrated that NGF is biosynthesized in peripheral tissues with sympathetic innervation and brain, and that fibroblasts or astroglial cells, which are interstitial cells in the peripheral or brain tissue, play an important role in the biosynthesis of NGF [J. Biol. Chem., 259:1259 (1984); Biochem. Biophys. Res. Commun., 136:57 (1986)]. It was also shown that the NGF produced by fibroblasts or astroglial cells had the same antigenicity, monocular weight, isoelectirc point and biological activity as those of submandibular gland NGF, which had been extensively studied. In addition, it was found that, when various neurotransmitters were added to cultures of fibroblasts (L-M cells) or astroglial cells, catecholamines (norepinephrine, epinephrine and dopamine) exhibited effects of promoting NGF synthesis [J. Biol. Chem., 201;6039 (1986); FEBS Lett., 208:258 (1986)].

Based on the above-mentioned findings, NGF is expected to be used as a therapeutic drug for preventing . the degeneration in nerve diseases in which the site on which NGF acts as a neurotrophic factor is degenerated. Once cerebral nerve cells are degenerated due to a cerebrovascular disease, brain tumor, apex of brain, a degenerative disease associated with head injury, anesthetic intoxication and the like, the cells will never be restored for life. As a result, various disorders such as lowered intellectual function and dysmnesia, as well as emotional disorder and abnormal behavior are caused. Nerve fibers have plasticity. When they are damaged, nerve fibers germinate from healthy fibers near the damaged site to form new synapses in place of the damaged synapses. It is expected that NGF can be used as a therapeutic drug for promoting repair and regeneration of the nerve function during this process.

If NGF is to be applied to treatment of various nerve diseases, NGF must reach the site in the very vicinity of nerve cells which require NGF. Also, in the case of diseases of the central nervous system, NGF must be delivered to affected cerebral cells. However, it is impossible to deliver NGF into brain through the vascular system. This is because vascular endothelial cells in the brain are stuck to each other (referred to as blood-brain barrier) to restrict transition of substances other than water, gas and lipid-soluble substances from the blood to the brain tissue, and thus macromolecules such as proteins (including NGF) cannot pass through the blood brain barrier at all. Thus, even if the current techniques are employed, it is too risky to administer NGF directly into the brain by surgical means.

For treatment or prevention of diseases to which growth factors are applicable, a method in which the growth factors are induced in vivo instead of administering them may be used. In this case, it would be possible to treat or prevent the diseases safely without abnormal side effects due to the administration of growth factors.

For example, if HGF can be induced at a desired site in vivo rather than administering it from the outside, diseases that require enhancement of HGF expression for their treatment or prevention (including hepatitis, cirrhosis and cholestasia in liver) may be effectively treated or prevented.

Furthermore, the delivery of NGF-inducing substances to NGF-producer cells in brain may lead to the application of successful results obtained in vitro to therapeutic drugs.

### Objects of the Invention

The main object of the present invention is to provide a highly safe compound derived from natural products having an ability to enhance growth factor production and/or interleukin-12 production, and to provide a pharmaceutical composition as well as a food and a drink useful for treating or preventing a disease that requires enhancement of growth factor production for its treatment or prevention.

### Summary of the Invention

The present invention is outlined as follows. The first aspect of the present invention relates to a pharmaceutical composition for treating or preventing a disease that requires enhancement of growth factor production for its treatment or prevention and/or a disease that requires enhancement of interleukin-12 production for its treatment or prevention, the pharmaceutical composition containing, as an active ingredient, a compound selected from the group consisting of 4,5-dihydroxy-2-cyclopenten-1-one of formula (I): 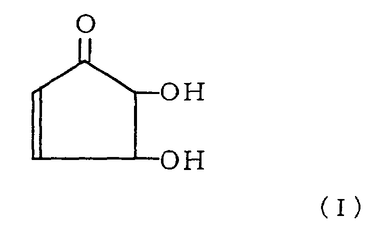 , 4-hydroxy-2-cyclopenten-1-one and derivatives thereof.

The second aspect of the present invention relates to a composition for enhancing growth factor production and/or interlukin-12 production containing, as an active ingredient, a compound selected from the group consisting of 4,5-dihydroxy-2-cyclopenten-1-one of formula (I), 4-hydroxy-2-cyclopenten-1-one and derivatives thereof.

The third aspect of the present invention relates to a food or a drink for enhancing growth factor production and/or interluekin-12 production which contains, which is produced by diluting and/or which is produced by adding thereto a substance selected from the group consisting of materials containing 4,5-dihydroxy-2-cyclopenten-1-one of formula (I), materials containing 4-hydroxy-2-cyclopenten-1-one, materials containing 4,5-dihydroxy-2-cyclopenten-1-one derivatives and materials containing 4-hydroxy-2-cyclopenten-1-one derivatives.

The present inventors have found that a compound selected from the group consisting of 4,5-dihydroxy-2-cyclopentenone-1-one of formula (I), 4-hydroxy-2-cyclopenten-1-one and derivatives thereof has an activity of enhancing growth factor production and/or an activity of enhancing interleukin-12 production, and is useful for enhancement of growth factor production and enhancement of interleukin-12 production. Thus, the present invention has been completed.

### Brief Description of Drawings

Figure 1 shows the relationship between the heating temperature and the amount of 4HCP produced.
Figure 2 shows the relationship between the heating time and the amount of 4HCP produced.
Figure 3 shows the relationship between the pH and the amount of 4HCP produced.
Figure 4 shows the relationship between the heating temperature and the amount of 4ACP produced.
Figure 5 shows the relationship between the heating temperature and the amount of 4GCP produced.
Figure 6 shows the relationship between the heating time and the amount of 4ACP produced.
Figure 7 shows the relationship between the heating time and the amount of 4GCP produced.
Figure 8 shows the relationship between the pH and the amount of 4ACP produced.
Figure 9 shows the relationship between the pH and the amount of 4GCP produced.
Figure 10 shows the relationship between the heating time and the amount of 4,5-dihydroxy-2-pentenal produced.

### Detailed Description of the Invention

The present invention will be more specifically explained below.

There is no particular limitation for a method of producing 4,5-dihydroxy-2-cyclopenten-1-one (hereinafter just referred to as cyclopentenone) used in the present invention. Cyclopentenone may be produced by a chemical synthesis method [Carbohydrate Res., 247:217-222 (1993); Helvetica Chimica Acta, 55:2838-2844 (1972)]. Furthermore, cyclopentenone produced in a heat treatment product of at least one substance selected from the group consisting of uronic acid, uronic acid derivatives, saccharides containing uronic acid and/or uronic acid derivatives, and materials which contain saccharides containing uronic acid and/or uronic acid derivatives as well as a product purified therefrom can be used. The heat treatment products containing cyclopentenone as well as products partially purified or purified therefrom can be used in the present invention. The above has been disclosed in WO 98/13328, of which the disclosure is herein incorporated by reference.

There is no particular limitation for materials containing cyclopentenone. In particular, the above-mentioned heat treatment products containing cyclopentenone and products partially purified therefrom can be preferably used in the third aspect of the present invention.

According to the present invention, a 4,5-dihydroxy-2-cyclopenten-1-one derivative (hereinafter just referred to as a cyclopentenone derivative) is not specifically limited as long as it is a derivative of cyclopentenone and has an ability to enhance growth factor production and/or interleukin-12 production. Examples thereof include cyclopentenone derivatives of formulas (II) to (V).

There is no limitation for a method of producing 4-hydroxy-2-cyclepenten-1-one (hereinafter just referred to as 4HCP) used in the present invention. 4HCP may be prepared by a chemical synthesis method. Known synthesis methods therefor include a method of Tanaka, T. et al. [Tetrahedron, 32:1713 (1976)], a method of Nara, M. et al. [Tetrahedron, 36:3161 (1980)] and a method of Gill, M. et al. [Aust. J. Chem., 34:2587 (1981)]. These production methods comprise many synthesis steps, are complicated, are of low yield, and are inefficient. 4HCP can -be obtained with high yield in one step by reducing 4-cyclopentene-1,3-dione with cerium (III) chloride and sodium boron hydride.

4HCP produced in a heat treatment product of at least one substance selected from the group consisting of pentose, pentose derivatives, compounds containing pentose and compounds containing pentose derivatives, as well as a product purified therefrom can be used. These heat treatment products containing 4HCP as well as products partially purified or purified therefrom can be used in the present invention. The above has been disclosed in WO 99/36383, of which the disclosure is herein incorporated by reference.

There is no particular limitation for materials containing 4HCP. In particular, the above-mentioned heat treatment products containing 4HCP and products partially purified therefrom can be preferably used in the third aspect of the present invention.

A 4HCP derivative is not specifically limited as long as it is a derivative of 4HCP and has an ability to enhance growth factor production and/or interleukin-12 production. Examples thereof include the derivatives of formulas (VI) and (VII). Furthermore, 4-(9-adeninyl)-2-cyclopenten-1-one (hereinafter referred to as 4ACP) and 4-(9-guaninyl)-2-cyclopenten-1-one (hereinafter referred to as 4GCP) produced in a heat treatment product of at least one substance selected from the group consisting of pentose, pentose derivatives, compounds containing pentose and compounds containing pentose derivatives, as well as products purified therefrom may be used. These heat treatment products containing 4ACP and/or 4GCP as well as products partially purified or purified therefrom can be used in the present invention.

These materials containing cyclopentenone derivatives and materials containing 4HCP derivatives may be used in the present invention as well. It is also possible to use optical isomers of cyclopentenone, cyclopentenone derivatives, 4HCP and 4HCP derivatives, as well as salts thereof. 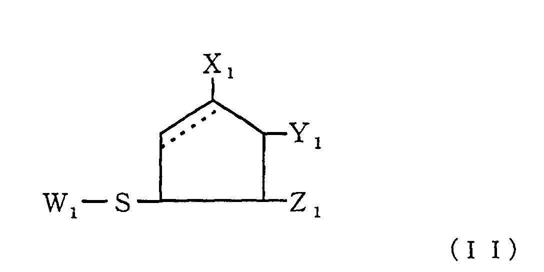 wherein a bond in the five-membered ring represented by a broken line means that the five-membered ring may be either a cyclopentene ring having a double bond or a saturated cyclopentane ring; in the case of a cyclopentene ring, X₁ is OH, Y₁ is =O and Z₁ is H; on the other hand, in the case of a cyclopentane ring, X₁ is =O, Y₁ is OH and Z₁ is OH; W₁ is a residue in which a SH group is removed from a SH group-containing compound. 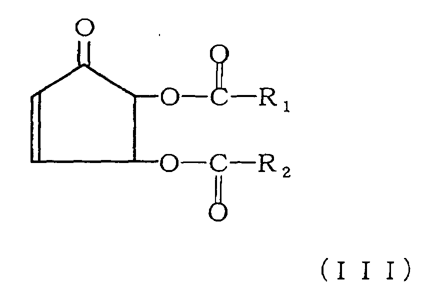 wherein R₁ and R₂ may be the same or different each other, and are hydrogen, or an aliphatic, aromatic or aromatic aliphatic group. 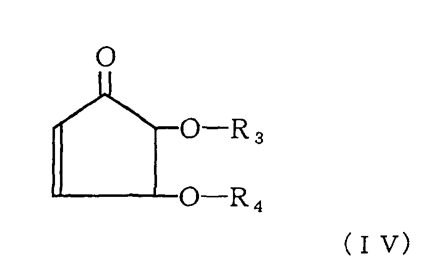 wherein R₃ and R₄ may be the same or different each other, and are hydrogen, or an aliphatic, aromatic or aromatic aliphatic group, provided that R₃ and R₄ are not simultaneously H. 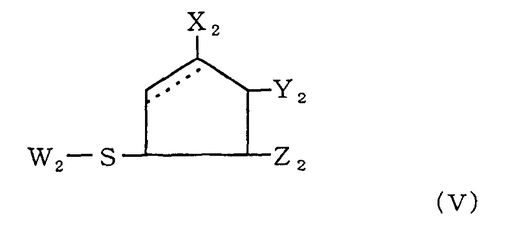 wherein a bond in the five-membered ring represented by a broken line means that the five-membered ring may be either a cyclopentene ring having a double bond or a saturated cyclopentane ring; in the case of a cyclopentene ring, X₂ is OR₅, Y₂ is =O and Z₂ is H; on the other hand, in the case of a cyclopentane ring, X₂ is =O, Y₂ is OR₆ and Z₂ is OR₇; R₅ is R₈ or -(CO)-R₉; R₆ is H, R₁₀ or -(CO)-R₁₁; and R₈ is H, R₁₂ or -(CO)-R₁₃ (wherein R₈, R₉, R₁₀, R₁₁, R₁₂ and R₁₃ may be the same or different each other, and are an aliphatic, aromatic or aromatic aliphatic group, and R₉, R₁₁ and R₁₃ may be H), provided that R₆ and R₇ are not simultaneously H; W₂ is a residue in which a SH group is removed from a SH group-containing compound. 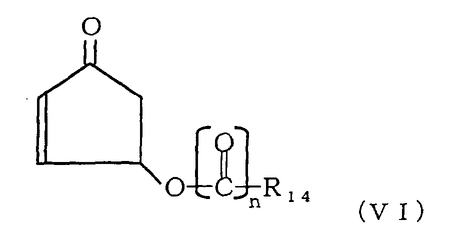 wherein R₁₄ is an aliphatic, aromatic or aromatic aliphatic group, and n is 0 or 1, provided that if n is 0, R₁₄ is not H. 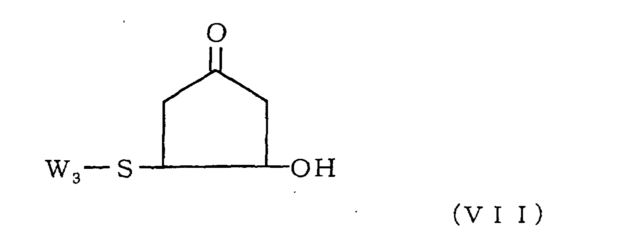 wherein W₃ is a residue in which a SH group is removed from a SH group-containing compound.

The cyclopentenone derivative of formula (II) is described in detail in WO 98/39291. The derivative can be obtained by reacting cyclopentenone with a SH group-containing compound such as cysteine or glutathione. Also, the material containing cyclopentenone derivative can be obtained by adding a SH group-containing compound to a material containing cyclopentenone. Examples of the derivatives include compounds of formulas (VIII) to (XI). Hereinafter, the compound of formula (VIII) is just referred to as GM.

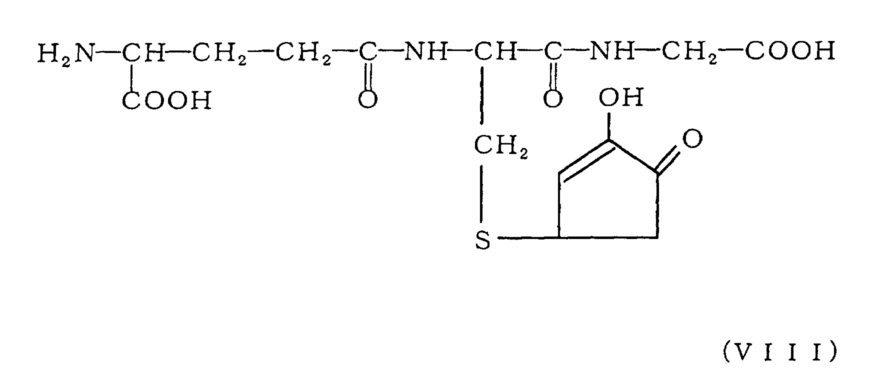

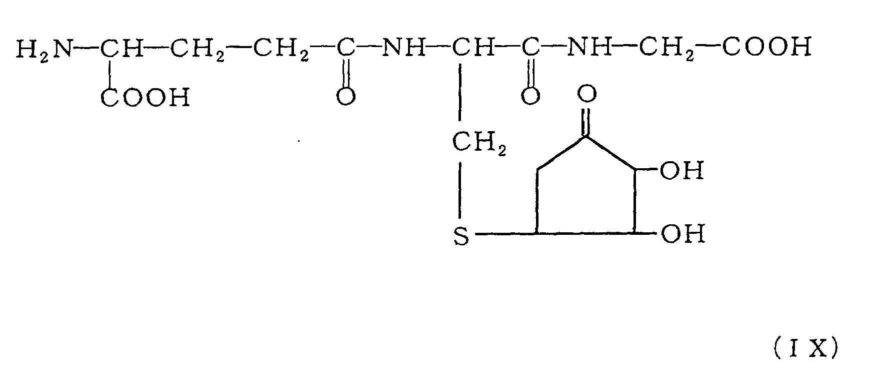

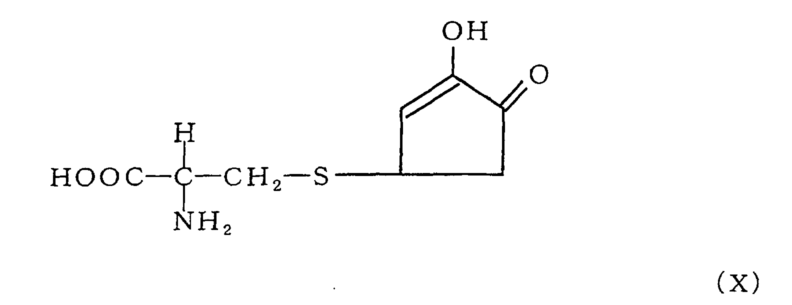

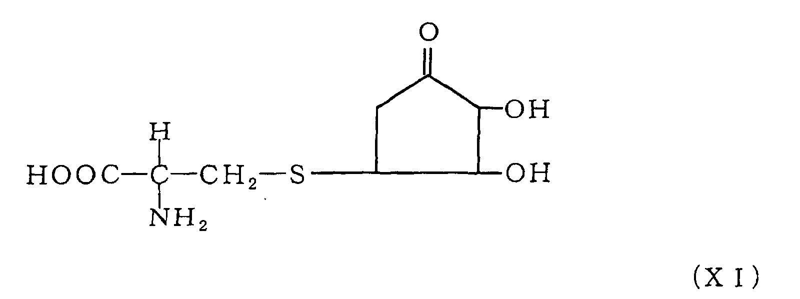

The cyclopentenone derivative of formula (III) is described in detail in WO 98/40346 and PCT/JP99/04323. The derivative can be obtained by simultaneously or successively reacting cyclopentenone with carboxylic acid having an aliphatic, aromatic or aromatic aliphatic group and/or its reactive derivative. Examples of the cyclopentenone derivatives include diacetylcyclopentenone, dibenzoylcyclopentenone, dihexanoylcyclopentenone, dimyristoylcyclopentenone, dioctanoylcyclopentenone, di-3-octenoylcyclopentenone, dibutyrylcyclopentenone, didecanoylcyclopentenone, divalerylclopentenone, dipropionylcyclopentenone, di-2-hexanoylcyclopentenone, diisobutylylcyclopentenone, dimethoxyacetylcyclopentenone, methoxyfumarylcyclopentenone, methoxymaleylcyclopentenone. Formula (XII) represents the structure of dipropionylcyclopentenone. Formula (XIII) represents the structure of dibenzoylcyclopentenone.

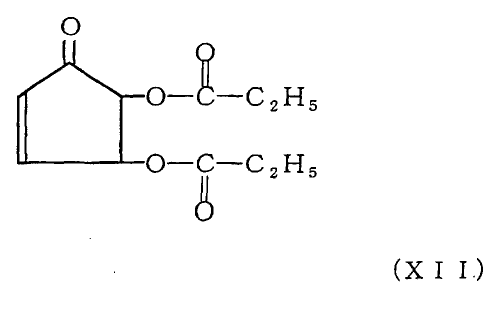

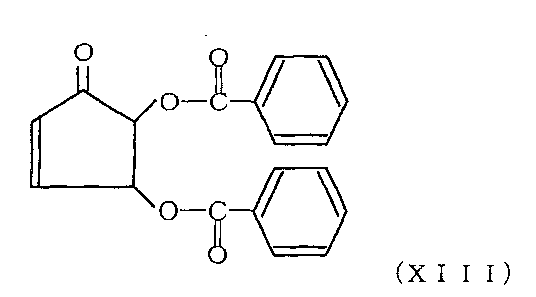

The cyclopentenone derivative of formula (IV) is described in detail in WO 99/00349. The derivative can be obtained by simultaneously or successively reacting cyclopentenone with alcohol having an aliphatic, aromatic or aromatic aliphatic group and/or its reactive derivative. Examples of the cyclopentenone derivatives include 4-benzylcyclopentenone ether, 5-benzylcyclopentenone ether, 4,5-benzylcyclopentenone ether, 4-t-butylcyclopentenone ether, 5-t-butylcyclopentenone ether, 4,5-di-t-butylcyclopentenone ether. Formulas (XIV) and (XV) represent the structures of 5-benzylcyclopentenone ether and 4,5-di-t-butylcyclopentenone ether, respectively.

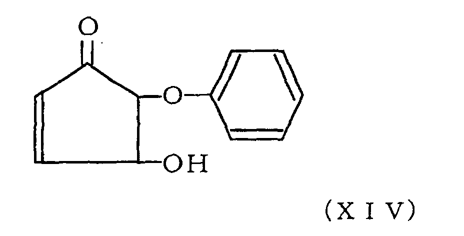

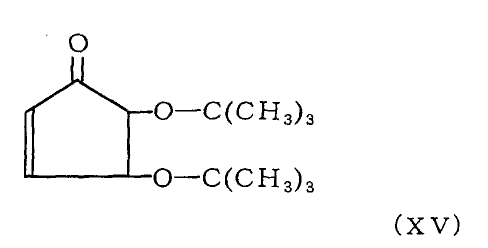

The cyclopentenone derivative of formula (V) is described in detail in PCT/JP99/04324. Substances containing the cyclopentenone derivative can be obtained by reacting a compound of formula (III) or (IV) with a SH group-containing compound such as cysteine or glutathione. Examples of the cyclopentenone derivatives include compounds of formulas (XVI) and (XVII).

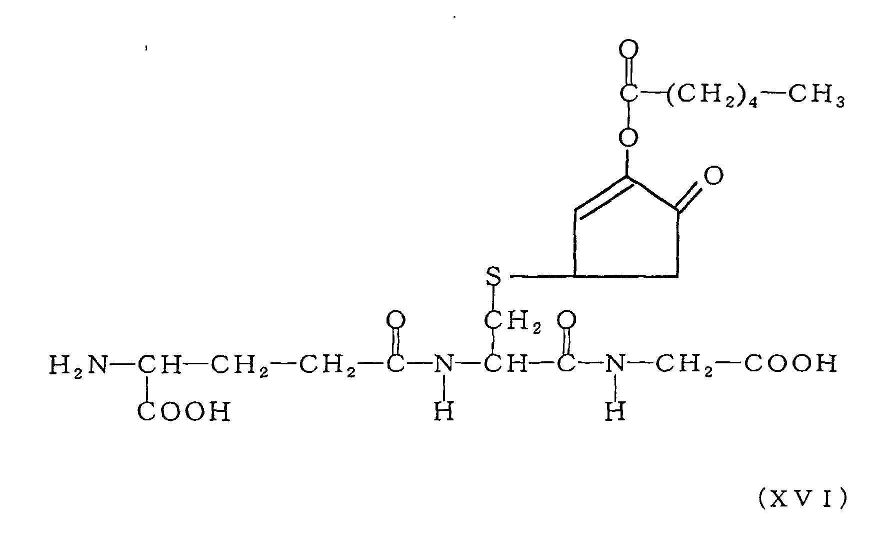

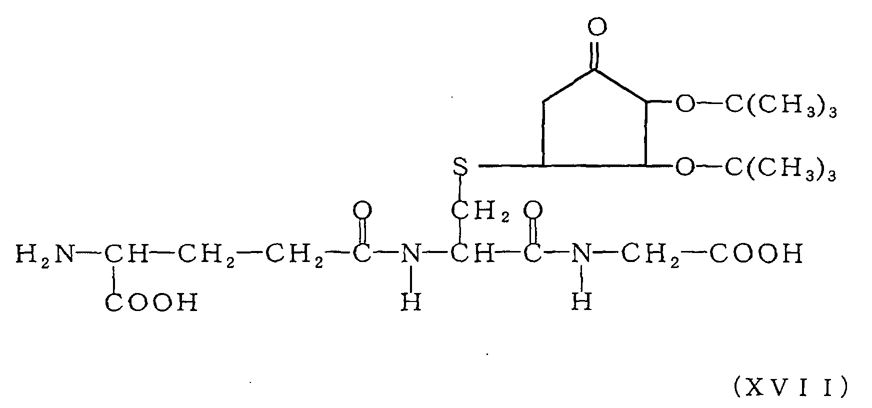

The 4HCP derivative of formula (VI) can be obtained by reacting 4HCP with carboxylic acid having an aliphatic, aromatic or aromatic aliphatic group and/or its reactive derivative, or by reacting 4HCP with alcohol having an aliphatic, aromatic or aromatic aliphatic group and/or its reactive derivative.

The 4HCP derivative of formula (VII) can be obtained by reacting 4HCP with a SH group-containing compound such as cysteine or glutathione, as described in WO 99/36383. Also, a material containing 4HCP derivative can be obtained by adding a SH group-containing compound to a material containing 4HCP. Examples the derivatives include the compound of formula (XVIII).

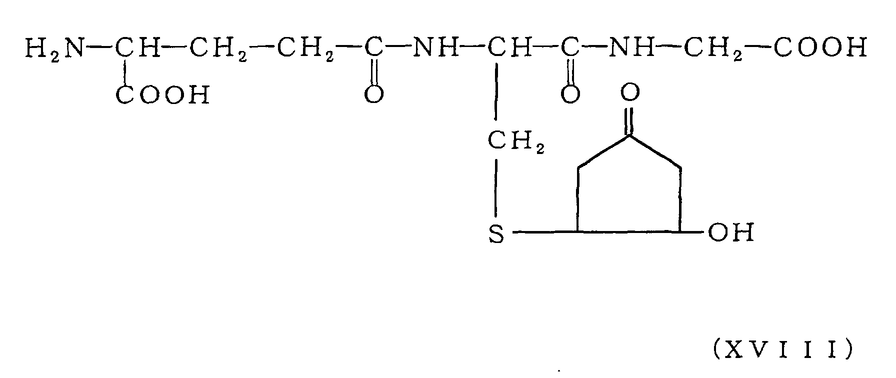

In the present invention, there is no particular limitation for the growth factors for which the enhancement of production is required, as long as they have activities of promoting cell growth. Examples thereof include HGF, NGF, epidermal growth factor, milk-derived growth factor, fibroblast growth factor, brain-derived fibroblast growth factor, acidic fibroblast growth factor, platelet-derived growth factor, platelet basic protein, connective tissue-activating peptide, insulin-like growth factor, colony stimulating factors, erythropoiethin, thrombopoietin, T cell growth factor, interleukins (e.g., interleukin-2, 3, 4, 5, 7, 9, 11 or 15), B cell growth factor, cartilage-derived factor, cartilage-derived growth factor, bone-derived growth factor, skeletal growth factor, endothelial cell growth factor, endothelial cell-derived growth factor, eye-derived growth factor, testis-derived growth factor, Sertoli cell-derived growth factor, mammotropic factor, spinal cord-derived growth factor, macrophage-derived growth factor, recycled mesodermal growth factor, transforming growth factor-α, transforming growth factor-β, heparin-binding EGF-like growth factor, amphiregulin, SDGF, betacellulin, epiregulin, neuregulins 1, 2, and 3, vascular endothelial growth factor, neurotrophin, BDNF, NT-3, NT-4, NT-5, NT-6, NT-7, glial cell line-derived neurotrophic. factor, stem cell factor, midkine, pleiotrophin, ephrin, angiopoietin, activin, tumor necrosis factor and interferons.

HGF exhibits an activity of proliferating hepatocyte, an activity of promoting protein synthesis, an activity of improving cholestasia, an activity of preventing renal damage due to drug and the like. mRNA for HGF is synthesized in brain, kidney, lung and the like. HGF exhibits activities of proliferating hepatocytes, renal tubule cells, epidermal cells and the like. Thus, HGF is a mesodermic cell growth factor. Accordingly, by enhancing hepatocyte growth factor production, it is possible to treat or prevent hepatitis, severe hepatitis, cirrhosis, cholestasia in liver, chronic nephritis, pneumonia and wound.

NGF is an endogenous growth factor that maintains survival and functions of nerve cells or elongates nerve cells according to a concentration gradient of NGF. Thus, by enhancing NGF production, it is possible to treat or prevent diseases that require repair/regeneration of the neurologic function for their treatment or prevention, e.g., senile dementia such as Alzheimer's disease, peripheral neuropathy, a cerebrovascular disease, brain tumor, apex of brain, a degenerative disease associated with head injury, anesthetic intoxication and the like.

Insulin-like growth factor exhibits various physiological activities against various cells. It is possible to treat or prevent type II diabetes (non-insulin dependent diabetes mellitus) and growth impairment (dwarfism) by enhancing insulin-like growth factor production. Furthermore, insulin-like growth factor promotes regeneration of sciatic nerve in sciatic nerve impairment. Thus, it may be used to treat or prevent amyotrophic lateral sclerosis.

Cyclopentenone, cyclopentenone derivatives, 4HCP and 4HCP derivatives exhibit abilities of enhancing interleukin-12 production. Therefore, there is provided a composition for enhancing interleukin-12 production containing, as an active ingredient, a compound selected from the group consisting of cyclopentenone, cyclopentenone derivatives, 4HCP and 4HCP derivatives. Said composition can be formulated by a conventional method and used as a pharmaceutical composition for treating or preventing a disease that requires enhancement of interleukin-12 production for its treatment or prevention.

Interferonγ (IFNγ) is produced when T cells (Th1) are stimulated by antigen presenting cells (APCs) or the like through T cell receptors (TCR). The production is further induced by interleukin-12 (IL-12), which is produced from the APCs. Thus, the production of IFNγ is induced by enhancing IL-12 production. The induced IFNγ activates cellular immunity (involving cytotoxic T cells, NK cells, macrophages and the like) against viral infections, fungal infections, cancerous diseases and the like to enhance the ability of biological defense. On the other hand, it is considered that suppression of the activation of Th2, which is considered to cause allergic diseases, may be effective for treating or preventing asthma, pollinosis, atopic dermatitis and the like.

Furthermore, cyclopentenone, cyclopentenone derivatives, 4HCP and 4HCP derivatives have activities of increasing/strengthening muscle. Therefore, there is provided a composition for increasing/strengthening muscle containing one of these compounds or materials containing the compounds as an active ingredient. There is no particular limitation for the materials containing the compounds. For example, a heat treatment product of DNA which contains a large amount of 4HCP can be used. The heat treatment product is obtained by heating DNA, which is a compound containing pentose.

The activity of increasing/strengthening muscle can be determined by evaluating a degree of recovery of muscle contracted by crushing sciatic nerves in terms of the function and the amount. It has been reported that the recovery from the impairment in this model experiment is largely due to regeneration of nerves. Vitamin B1 and vitamin B12 were reported to strikingly promote the elongation of nerve fibers in experiments of tissue culture of fetal chicken sensory ganglion or spinal cord, and also have significant effects of recovering the quantity of muscle (e.g., soleus muscle) in a sciatic nerve crush model (Folia pharmacol. Japon. 74:721-734 (1978)).

The heat treatment product of DNA has not been reported to be effective in the model experiments as described above. Its effect of enhancing the production of insulin-like growth factor, HGF or NGF, or of increasing muscle or recovering a function of muscle has not been reported. The present inventors found that the heat treatment product of DNA enhances the production of insulin-like growth factor, HGF and NGF. Thus, it can be used for treatment or prevention of not only amyotrophic lateral sclerosis but also growth impairment, osteoporosis, diabetes and renal insufficiency which require insulin-like growth factors for their treatment or prevention.

A compound selected from the group consisting of cyclopentenone, cyclopentenone derivatives, 4HCP and 4HCP derivatives has an ability to enhance growth factor production and/or interleukin-12 production. Thus, a pharmaceutical composition for treating or preventing a disease that requires enhancement of growth factor production for its treatment or prevention and/or a disease that requires enhancement of interleukin-12 production for its treatment or prevention can be produced using one of these compounds as an active ingredient. It is also possible to produce a composition for increasing/strengthening muscle using the compound as an active ingredient.

The pharmaceutical composition for treatment or prevention can be formulated by using a compound selected from the group consisting of cyclopentenone, cyclopentenone derivatives, 4HCP and 4HCP derivatives as its active ingredient, and formulating it with a known pharmaceutical carrier. The composition is generally mixed with a pharmaceutically acceptable liquid or solid carrier and, optionally, solvent, dispersing agent, emulsifier, buffering agent, stabilizer, excipient, binder, disintegrant, lubricant and the like to formulate it. The formulation may be in a form of a solid preparation such as tablet, granule, powder, epipastic and capsule, or a liquid preparation such as normal solution, suspension and emulsion. In addition, it may be formulated into a dried preparation, which can be reconstituted as a liquid preparation by adding an appropriate carrier before use.

The pharmaceutical composition for treatment or prevention can be administrated as either an oral preparation or a parenteral preparation such as injectable preparation and drips.

The pharmaceutical carrier can be selected according to the above-mentioned particular administration route and dosage form. For an oral preparation, starch, lactose, sucrose, mannit, carboxymethylcellulose, cornstarch, inorganic salts and the like are utilized, for example. Binder, disintegrant, surfactant, lubricant, fluidity-promoting agent, tasting agent, coloring agent, flavoring agent and the like can also be included in oral preparations.

A parenteral preparation can be prepared according to conventional methods by dissolving or suspending a compound selected from the group consisting of cyclopentenone, cyclopentenone derivatives, 4HCP and 4HCP derivatives as an active ingredient of the pharmaceutical composition for treatment or prevention in a diluent. The diluents include injectable distilled water, physiological saline, aqueous glucose solution, injectable vegetable oil, sesame oil, peanut oil, soybean oil, corn oil, propylene glycol and polyethylene glycol. Optionally, sterilizer, stabilizer, osmotic regulator, smoothing agent and the like may be added to the solution or suspension.

The pharmaceutical composition for treatment or prevention of the present invention is administered through a suitable route for the dosage form of the composition. The administration route is not limited to a specific one. The composition can be administered internally or externally (or topically) or by injection. The injectable preparation can be administrated intravenously, intramuscularly, subcutaneously, intradermally and the like, for example. External preparations include a suppository.

A dosage of the pharmaceutical composition for treatment or prevention is appropriately determined and varies depending on the particular dosage form, administration route and purpose as well as age, weight and conditions of a patient to be treated. In general, a daily dosage for an adult person is 10 µg to 200 mg/kg in terms of the amount of the active ingredient contained in the formulation. Of course, the dosage can vary depending on various factors. Therefore, in some cases, a less dosage than the above may be sufficient but, in other cases, a dosage more than the above may be required. The pharmaceutical composition of the present invention can be administrated orally as it is, or it can be taken daily by adding to selected foods and drinks.

The composition for enhancing growth factor production or interleukin-12 production containing, as an active ingredient, a compound selected from the group consisting of cyclopentenone, cyclopentenone derivatives, 4HCP and 4HCP derivatives of the present invention may be produced and administered by a method similar to those described above for the pharmaceutical composition for treatment or prevention. The composition for enhancing growth factor production or interleukin-12 production may be used in a method for enhancing growth factor production, and is useful for studying the function of growth factors, and for screening medicines related to growth factors.

There is no particular limitation for the composition for enhancing growth factor production of the present invention. Examples thereof include a composition for enhancing HGF production, a composition for enhancing NGF production, and a composition for enhancing h-IGF production, which can be used as a pharmaceutical composition for treating or preventing a disease that requires enhancement of HGF production, a pharmaceutical composition for treating or preventing a disease that requires enhancement of NGF production, and a pharmaceutical composition for treating or preventing a disease that requires enhancement of h-IGF production for its treatment or prevention, respectively.

A compound selected from the group consisting of cyclopentenone, cyclopentenone derivatives, 4HCP and 4HCP derivatives is used as the active ingredient of the composition for enhancing HGF production of the present invention. Although it is not intended to limit the present invention, a compound having a cyclopentene ring is preferably used. Also, the composition for enhancing HGF production of the present invention may contain a substance that induces the transcription of HGF gene to enhance the production of HGF, the substance being selected from the group consisting of cytokines and prostaglandins including IL-1, prostaglandin E₁ and prostaglandin E₂. If the composition for enhancing HGF production of the present invention acts to induce the transcription of HGF gene, it is preferable that the composition includes a substance having an activity of promoting the step of the translation of mRNA for HGF gene or downstream steps to enhance HGF production. Then, there is provided a composition for enhancing HGF production by which HGF production is synergistically enhanced. There is no particular limitation for the substance used for exerting the synergistic effect as long as it has an activity of promoting the step of the translation of mRNA for HGF gene or downstream steps to enhance HGF production. Examples thereof include sulfated polysaccharide such as heparin, heparan sulfate and fucoidan as well as degradation products thereof. Shogaol or gingerol derived from ginger, curcumin derived from curcuma, or the like, which has an activity of enhancing HGF production, or materials containing these substances may be contained in the composition for enhancing HGF production of the present invention. Furthermore, the composition for enhancing growth factor production and/or interleukin-12 production of the present invention may be taken daily by adding to selected foods and drinks.

The composition for increasing/strengthening muscle containing, as an active ingredient, a compound selected from the group consisting of cyclopentenone, cyclopentenone derivatives, 4HCP and 4HCP derivatives of the present invention may be produced and administered by a method similar to those described above for the pharmaceutical composition for treatment or prevention.

There is no particular limitation for the method for producing a food or a drink for enhancing growth factor production and/or interleukin-12 production which contains, which is produced by diluting and/or which is produced by adding thereto a substance as an active ingredient selected from the group consisting of materials containing cyclopentenone, materials containing cyclopentenone derivatives, materials containing 4HCP and materials containing 4HCP derivatives. Any processes including cooking, processing and other generally employed processes for producing foods and drinks can be used as long as the resultant food or drink contains an effective amount of a substance having a physiological activity selected from the group consisting of materials containing cyclopentenone, materials containing cyclopentenone derivatives, materials containing 4HCP and materials containing 4HCP derivatives. The food or drink may be taken as a functional food or drink having an activity of enhancing growth factor production and/or interleukin-12 production.

Also, there is no particular limitation for the method for producing a food or a drink for increasing/strengthening muscle, which contains, which is produced by diluting and/or which is producing by adding thereto a substance as an active ingredient selected from the group consisting of materials containing cyclopentenone, materials containing cyclopentenone derivatives, materials containing 4HCP and materials containing 4HCP derivatives. Any processes including cooking, processing and other generally employed processes for producing foods and drinks can be used as long as the resultant food or drink contains an effective amount of a substance having a physiological activity selected from the group consisting of materials containing cyclopentenone, materials containing cyclopentenone derivatives, materials containing 4HCP and materials containing 4HCP derivatives. The food or drink may be taken as a functional food or drink having an activity of increasing/strengthening muscle.

When cyclopentenone, a cyclopentenone derivative, 4HCP or a 4HCP derivative used in the present invention is administered in an physiologically effective amount, no toxicity is observed. Also, no toxicity is observed for the materials containing these substances.

For example, no example of death was observed when a single dose of cyclopentenone, GM, 4HCP, dipropionylcyclopentenone, dihexanoylcyclopentenone, di-2-hexenoylcyclopentenone, diisobutylcyclopentenone, dibenzoylcyclopentenone, 4-t-butylcyclopentenone ether or 4,5-di-t-butylcyclopentenone ether, or an optical isomer or a salt thereof was orally administered to a mouse at 100 mg/kg.

### Examples

The present invention will be further illustrated in detail by way of the following examples. However, the present invention is never limited to those examples. "%" used in the examples means "% by weight".

### Referential Example 1

(1) 10 g of D-glucuroic acid (Sigma, G 5269) was dissolved in 1 1 of water. The solution was heated at 121°C for 4 hours and then concentrated to about 10 ml under reduced pressure. 40 ml of an upper layer of a mixture of butyl acetate : acetic acid : water = 3 : 2 : 2 was added thereto and mixed. The mixture was centrifuged to obtain a supernatant. The resultant supernatant was concentrated to about 10 ml under reduced pressure.
   The extract was applied to silica gel for column chromatography BW-300SP (2 x 28 cm; Fuji Silysia) and separated using an upper layer of butyl acetate : acetic acid : water = 3 : 2 : 2 as an eluent at a flow rate of 5 ml/minute while applying pressure at 0.2 kg /cm² using a compressor. Fractionation was carried out such that each fraction contained 10 ml of the eluate. A part of each fraction was analyzed on thin-layer chromatography. The 61st to 80th fractions contained highly pure cyclopentenone. Those fractions were collected, concentrated under reduced pressure, and then extracted with 40 ml of chloroform. The extract was concentrated under reduced pressure to obtain 100 mg of cyclopentenone.
   The fraction was separated on normal phase HPLC using a PALPAK type S column (Takara Shuzo). The purity was found to be 98% when detection was conducted using ultraviolet absorbance at 215 nm.
(2) 30 mg of cyclopentenone, 16 mg of dimethylaminopyridine (DMAP), 66 mg of triethylamirie and 86 mg of propionic anhydride (Tokyo Kasei Kogyo, PO513) were dissolved in 5.9 ml of dichloromethane. The mixture was reacted for 1 hour while cooling ice. Then, the reaction mixture was developed on silica gel thin-layer chromatography using chloroform : methanol = 200 : 1 as a developing solvent. Silica gel was scraped from a portion at Rf = 0.5 to 0.6 of the thin layer and extracted with chloroform to obtain 31 mg of dipropionylcyclopentenone.
   The results of structural analysis of the resultant dipropionylcyclopentenone by nuclear magnetic resonance are shown below.
   ¹H-NMR
   δ7.45 (1H, dd, J2-3=6.27 Hz, J3-4=2.15 Hz, H-3), 6.42 (1H, dd, J2-3=6.27 Hz, J3-4=1.49 Hz, H-2), 5.91 (1H, m, H-4), 5.16 (1H, d, J4-5=2.97 Hz, H-5), 2.46 (2H, dd, J=15.01, 7.59 Hz), 2.42 (2H, dd, J=15.01, 7.59 Hz), 1.18 (6H, dd, J=7.59, 7.59 Hz)
(3) 100 µl of a 1 M aqueous solution of cyclopentenone and 500 µl of a 200 mM aqueous solution of glutathione (reduced form: sold by Nacalai Tesque: 170-10) (pH3.0) were mixed together. The mixture was reacted at 60°C for 5 hours. The reaction mixture was filtered through 0.5 µm Cosmonice Filter (Nacalai Tesque), and separated on HPLC under the following conditions.
   Column: TSKgel ODS-80Ts (5 µm), 20 mm x 25 cm (Tosoh);
   Mobile Phase:
      A: 0.1% aqueous solution of trifluoroacetic acid (TFA);
      B: 0.1% TFA/50% aqueous solution of acetonitrile;
   Flow rate: 7.5 ml/minute;
   Gradient: Mobile Phase A (10 minutes) → Mobile Phase A to A : B = 1 : 1 (55 minutes) → Mobile Phase A : B = 1 : 1 to Mobile Phase B (15 minutes);
   Detection: Absorbance at 220 nm.

   200 µl of the reaction mixture was applied to HPLC. Peaks at retention time of 35.7 minutes and 36.1 minutes were collected, concentrated under reduced pressure to dryness to obtain 5.5 mg of a dry solid.
   The structure of the dry solid was analyzed. The nuclear magnetic resonance (NMR) spectrum and the mass spectrum (MS) were measured. The results are shown below.
   ¹H-NMR
   δ2.09 (2H, m, 5' -H), 2.28 (1H, dd, J=13.0, 20.0 Hz, 5-H), 2.44 (2H, m, 4' -H), 2.78 (1H, dd, J=8.5, 14.0, 1' -H), 2.85 or 2.89 (1H, dd, J=3.0, 6.0 Hz, 5-H), 2.92 or 2.95 (1H, dd, J=1.0, 5.5 Hz, 1'-H), 3.86 (2H, S, 9'-H), 3.95 (2H, m, 4-H, 6'-H), 4.46 (1H; m, 2'-H), 6.47 or 6.49 (1H, d, J=3.0 Hz, 3-H)

   The sample was dissolved in 0.1 N DCl solution in heavy water. The results are expressed assuming the chemical shift value of HOD as 4.65 ppm.
   ¹³C-NMR
   δ26.3 (5'-C), 31.7 (4'-C), 31.9 or 32.1 (1'-C), 39.3 (4-C), 41.9 (9'-C), 42.2 or 42.3 (5-C), 53.3 (6'-C), 54.1 (2'-C), 133.5 (3-C), 154.4 (2-C), around 173 (3'-C, 7'-C, 8'-C, 10'-C), 205.8 (1-C)

   The sample was dissolved in 0.1 N DCl solution in heavy water. The results are expressed assuming the chemical shift value of dioxane as 67.4 ppm.
   The numbers for peak identification in ¹H-NMR and ¹³C-NMR are as indicated in formula (XIX) below. 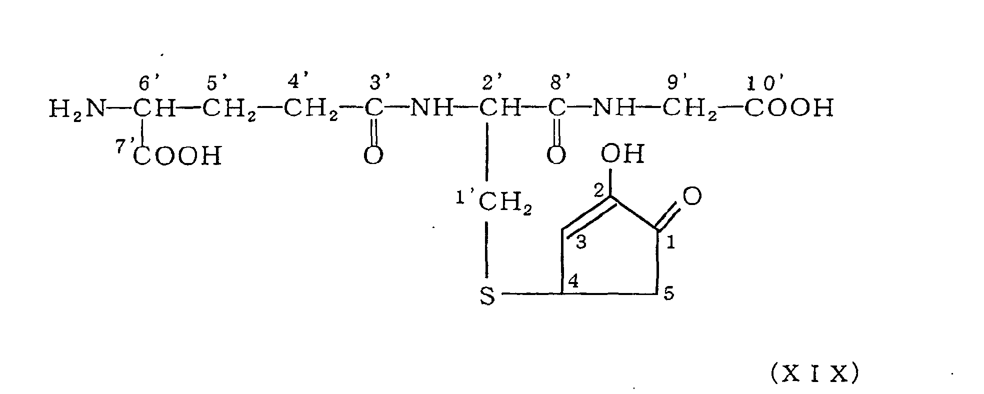
   FAB-MS
   m/z 404 (M+H)+, 426 (M+Na)+
   Glycerol was used for matrix.
   UV λmax 251 nm (water)
   IR νKBr max cm⁻¹ 2949, 1710, 1660, 1539, 1404, 1203

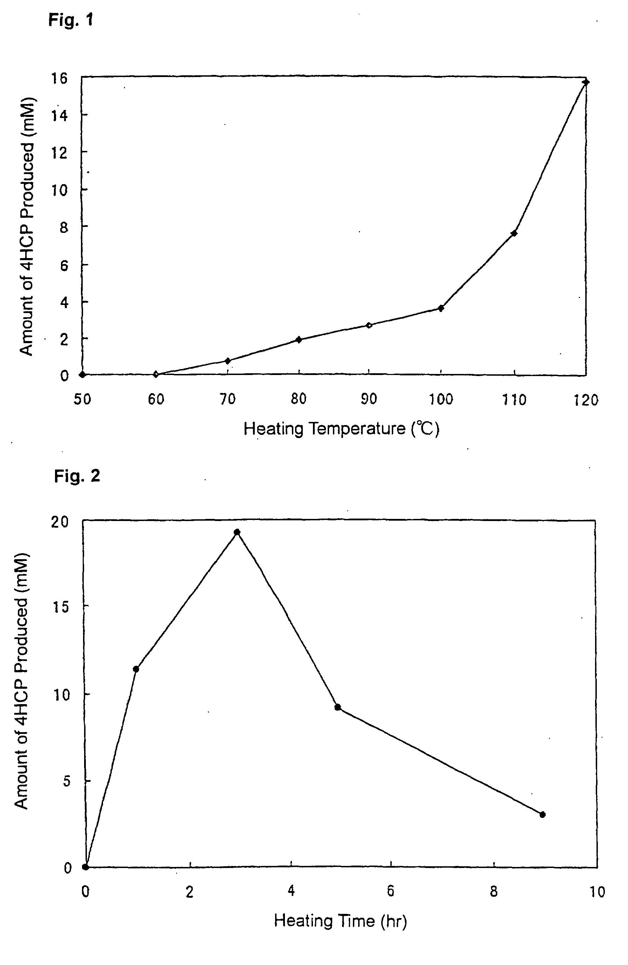

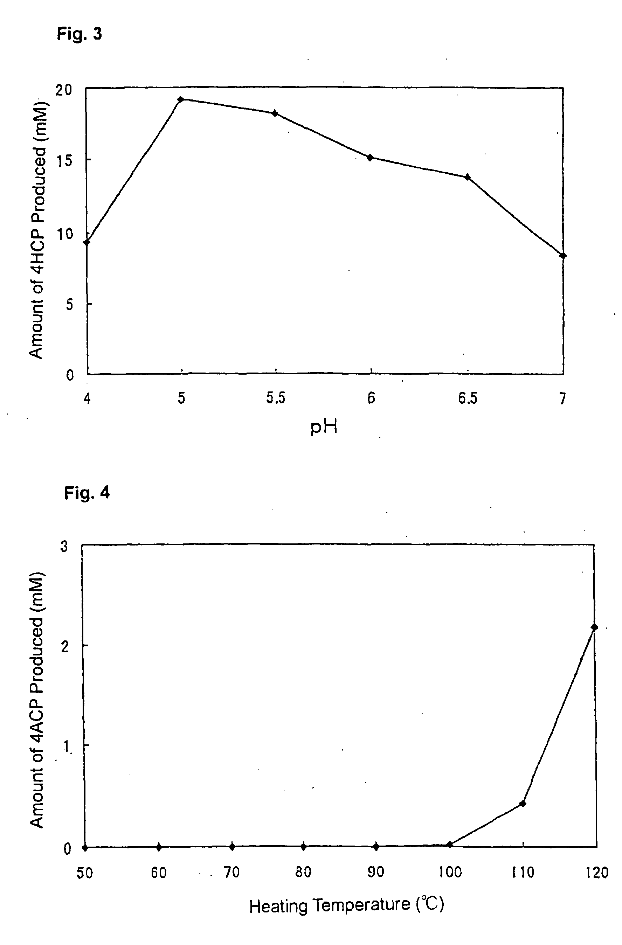

   Measurement was carried out according to diffuse reflectance method.
   The above-mentioned results showed that the dry solid was GM, i.e., 2-hydroxy-4-glutathion-S-yl-2-cyclopenten-1-one.
(4) A 5% (w/v) aqueous solution of DNA·Na (Nichiro) had a pH of 6.5. After the pH of the solution was adjusted to 5.5 by adding diluted HCl, the mixture was heated in a heat block for 3 hours at 50, 60, 70, 80, 90, 100, 110 or 120°C.
   The concentration of 4HCP in the supernatant of the heated mixture was measured by a thin-layer chromatography method.
   Briefly, 1 µl each of the supernatants of the heated mixtures as well as 2, 4, 6, 8, 10 and 12 mM aqueous solutions of 4HCP was spotted onto a silica gel plate for thin-layer chromatography (MERCK), and developed using a mixture of chloroform : methanol = 4 : 1. After red color was developed using an orcinol-sulfuric acid reagent, the image of the plate was read using Photo/Analyst Archiver (Fotodyne inc.), and the intensity of each spot was numerically expressed using 1-DBasic (Advanced American Biotechnology software) to determine the concentration in each sample using a calibration curve for 4HCP.
   The results are shown in Figure 1. Figure 1 shows the relationship between the heating time and the amount of 4HCP produced. In the figure, the vertical axis represents the amount of 4HCP produced (mM) and the horizontal axis represents the heating temperature (°C). As shown in the figure, the amount of 4HCP produced was increased as the heating temperature made higher.
(5) After pH of a 5% (w/v) aqueous solution of DNA·Na (Nichiro) was adjusted to 5.5 by adding diluted HCl, the mixture was heated for 0, 1, 3, 5 or 9 hours at 120°C using an autoclave.
   The concentration of 4HCP in the supernatant of the heated mixture was measured according to the method described in Referential Example 1-(4).
   The results are shown in Figure 2. Figure 2 shows the relationship between the heating time and the amount of 4HCP produced. In the figure, the vertical axis represents the amount of 4HCP produced (mM) and the horizontal axis represents the heating time (hour). As shown in the figure, the amount of 4HCP produced reached its peak at 3 hours of heating.
(6) After pH of a 5% (w/v) aqueous solution of DNA·Na (Nichiro) was adjusted to 4.0, 5.0, 5.5 or 6.0 by adding diluted HCl, or to 7.0 by adding diluted NaOH, the mixture was heated at 120°C using an autoclave for 3 hours.
   The concentration of 4HCP in the supernatant of the heated mixture was measured according to the method described in Referential Example 1-(4).
   The results are shown in Figure 3. Figure 3 shows the relationship between the pH and the amount of 4HCP produced. In the figure, the vertical axis represents the amount of 4HCP produced (mM) and the horizontal axis represents the pH. As shown in the figure, the increase in the amount of 4HCP production peaked at about pH 5.
(7) After pH of a 5% (w/v) aqueous solution of DNA•Na (Nichiro) was adjusted to 5.5 by adding diluted HCl, the mixture was heated in a heat block at 50, 60, 70, 80, 90, 100, 110 or 120°C for 3 hours.
   The supernatant of the heated mixture was analyzed on reversed phase HPLC under the following conditions.
   Column: YMCpack ODS-AM (ϕ 4.6 mm x 25 cm, YMC);
   Mobile Phase: 3% aqueous solution of acetonitrile, 0.8 ml/min.;
   Detection: 210 nm.

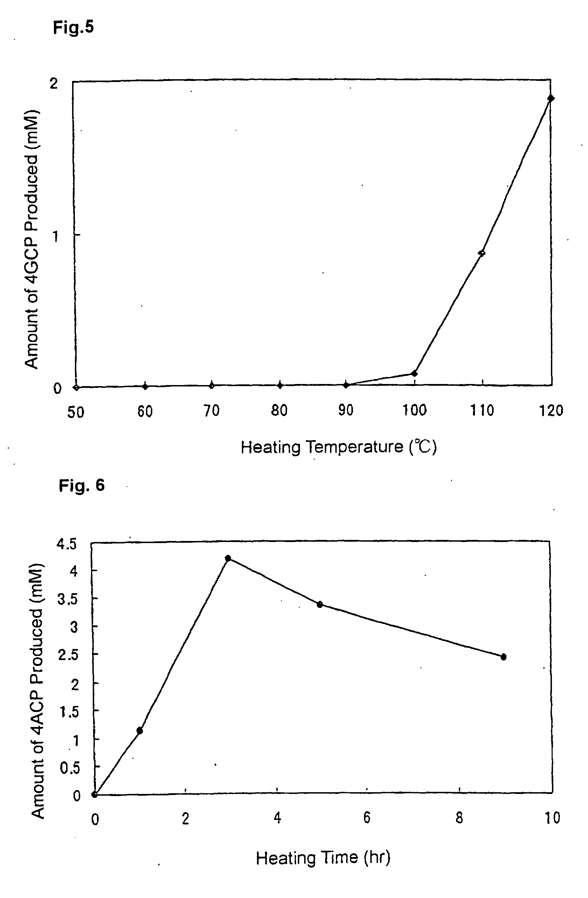

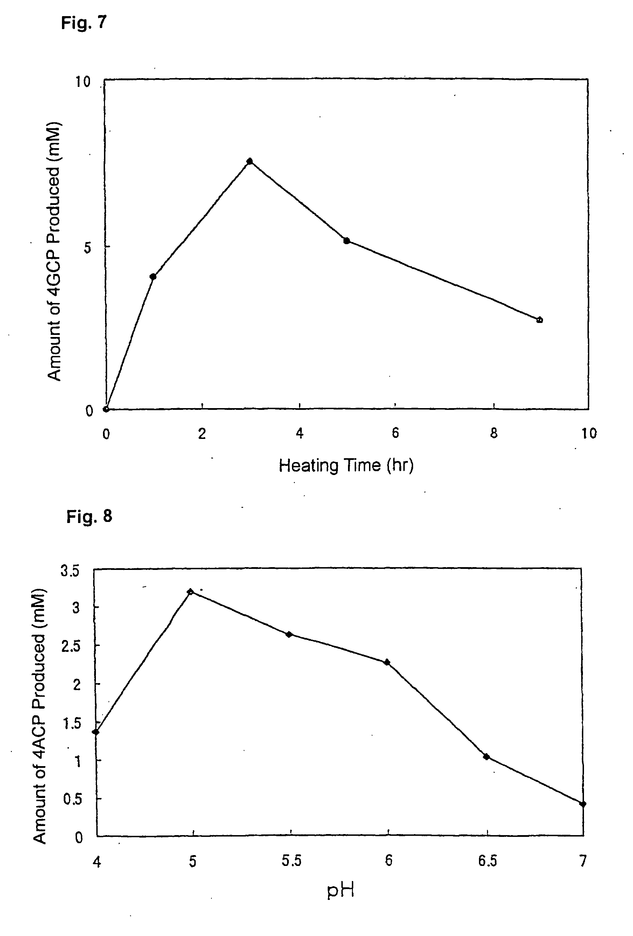

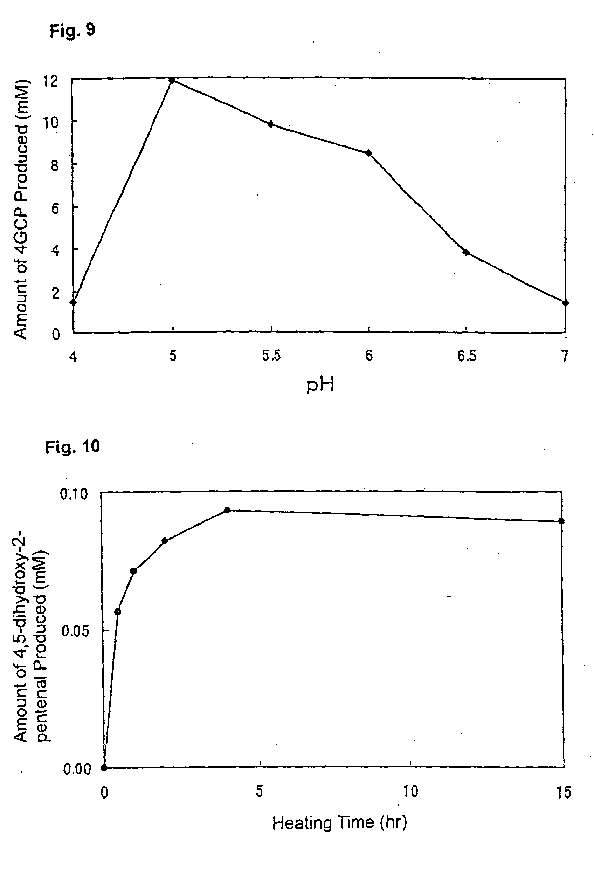

   Areas of peaks for 4GCP eluted at 25 minutes and for 4ACP eluted at 33 minutes were determined. Then, the concentrations of the respective compounds in the samples were determined using calibration curves for the respective compounds.
   The results are shown in Figures 4 and 5. Figure 4 shows the relationship between the heating temperature and the amount of 4ACP produced. In the figure, the vertical axis represents the amount of 4ACP produced (mM) and the horizontal axis represents the heating temperature (°C). Figure 5 shows the relationship between the heating temperature and the amount of 4GCP produced. In the figure, the vertical axis represents the amount of 4GCP produced (mM) and the horizontal axis represent the heating temperature (°C).
   As shown in Figures 4 and 5, the generation of 4ACP and 4GCP was observed when heated for 3 hours at 100°C or above, and a larger production amount was achieved by heating at a higher temperature.
(8) After pH of a 5% (w/v) aqueous solution of DNA•Na (Nichiro) was adjusted to 5.5 by adding diluted HCl, the mixture was heated at 120°C using an autoclave for 0, 1, 3, 5 or 9 hours.
   The concentrations of 4ACP and 4GCP in the supernatants of the heated mixtures were measured according to the method described in Referential Example 1-(7).
   The results are shown in Figures 6 and 7. Figure 6 shows the relationship between the heating time and the amount of 4ACP produced. In the figure, the vertical axis represents the amount of 4ACP produced (mM) and the horizontal axis represents the heating time (hour). Figure 7 shows the relationship between the heating time and the amount of 4GCP produced. In the figure, the vertical axis represents the amount of 4GCP produced (mM) and the horizontal axis represents the heating time (hour).
   As shown in Figures 6 and 7, the amounts of 4ACP and 4GCP reached their peaks at 3 hours, and decreased thereafter.
(9) After pH of a 5% (w/v) aqueous solution of DNA·Na (Nichiro) was adjusted to 4.0, 5.0, 5.5 or 6.0 by adding diluted HCl, or to 7.0 by adding diluted NaOH, the mixture was heated at 120°C using an autoclave for 3 hours.
   The concentrations of 4ACP and 4GCP in the supernatants of the heated mixtures were determined according to the method described in Referential Example 1-(7).
   The results are shown in Figures 8 and 9. Figure 8 shows the relationship between the pH and the amount of 4ACP produced. In the figure, the vertical axis represents the amount of 4ACP produced (mM) and the horizontal axis represents the pH. Figure 9 shows the relationship between the pH and the amount of 4GCP produced. In the figure, the vertical axis represents the amount of 4GCP produced (mM) and horizontal axis represents the pH.
   As shown in Figures 8 and 9, the increase in the amounts of both 4ACP and 4GCP peaked at about pH 5.
(10) A 0.1 M aqueous solution of 2-deoxy-D-ribose (Sigma) was heated at 121°C for 0.5, 1, 2, 4 or 15 hours.
   The supernatant of the heated solution was analyzed on reversed phase HPLC under the following conditions.
   Column: YMCpack ODS-AM (ϕ 4.6 mm x 25 cm, YMC);
   Mobile Phase:
      A: 0.1% aqueous solution of TFA;
      B: 0.1% TFA/80% aqueous solution of acetonitrile;
   Flow Rate: 0.8 ml/min.;
   Elution: Mobile Phase A (5 minutes) → linear gradient from Mobile Phase A to B (20 minutes) → Mobile Phase B;
   Detection: 215 nm.

   Area of the peak for 4,5-dihydroxy-2-pentenal eluted at 7 minutes was determined to determine the concentration in each sample using a calibration curve.
   The results are shown in Figure 10. Figure 10 shows the relationship between the heating time and the amount of 4,5-dihydroxy-2-pentenal produced. In the figure, the vertical axis represents the amount of 4,5-dihydroxy-pentenal produced (mM) and the horizontal axis represents the heating time (hour). As shown in Figure 10, the amount of 4,5-dihyoxy-2-pentenal production reached the plateau after heating for 5 hours.

### Example 1

Rat fibroblast L-M cells (ATCC CCL-1.2) were suspended in a M199 medium (Gibco) containing 0.5% Bacto Peptone (Gibco) at a concentration of 1.0 x 10⁵ cells/ml. 1 ml of the suspension was inoculated into each well of a 24-well plate. The cells were cultured aseptically. After culturing for 2 days, the medium was removed and substituted with a M199 medium containing 0.5% bovine serum albumin (Sigma). Then, cyclopentenone (final concentration of 1 to 25 µM), 4HCP (Aldrich) (final concentration of 12.5µM) or dipropionylcyclopentenone (final concentration of 3.1 to 9.4 µM) was added thereto. The plate was then incubated for 24 hours.

After completion of the incubation, the concentration of nerve growth factor in the culture was measured by enzyme immunoassay (NGF Emax Immuno Assay System: Promega). As a result, cyclopentenone, 4HCP and dipropionylcyclopentenone enhanced the production of nerve growth factor by L-M cells in a concentration-dependent manner. The results are shown in Tables 1 to 4.

**Table 1:**

| Enhancement of nerve growth factor production by cyclopentenone | |
|---|---|
| Cyclopentenone concentration (µM) | Nerve growth factor concentration (ng/ml) |
| 0 | 0.570 |
| 1 | 0.700 |
| 2.5 | 0.740 |
| 5 | 0.870 |
| 7.5 | 0.900 |
| 10 | 1.080 |
| 12.5 | 1.340 |
| 15 | 1.550 |
| 17.5 | 2.150 |
| 20 | 1.900 |
| 25 | 1.540 |

**Table 2:**

| Enhancement of nerve growth factor production by 4HCP | |
|---|---|
| 4HCP concentration (µM) | Nerve growth factor concentration (ng/ml) |
| 0 | 0.138 |
| 12.5 | 0.327 |

**Table 3:**

| Enhancement of nerve growth factor production by dipropionylcyclopentenone | |
|---|---|
| Dipropionylcyclopentenone concentration (µM) | Nerve growth factor concentration (ng/ml) |
| 0 | 0.132 |
| 3.1 | 0.164 |
| 6.3 | 0.259 |
| 9.4 | 0.257 |

**Table 4:**

| Enhancement of nerve growth factor production by cyclopentenone (time course) | | | | | | |
|---|---|---|---|---|---|---|
| Cyclopentenone | Cultivation time (hour). | | | | | |
| Concentration (µM) | 0 | 3 | 6 | 12 | 24 | 48 |
| | Nerve growth factor concentration (ng/ml) | | | | | |
| 0 | 0.000 | 0.028 | 0.233 | 0.575 | 0.658 | 0.736 |
| 15 | 0.000 | 0.050 | 0.359 | 0.686 | 1.186 | 1.236 |
| 17.5 | 0.000 | 0.054 | 0.205 | 0.635 | 1.535 | 1.492 |
| 20 | 0.000 | 0.082 | 0.179 | 0.581 | 1.681 | 1.874 |

Furthermore, other cyclopentenone derivatives enhanced the production of nerve growth factors by L-M cells as well.

### Example 2

MRC-5 cells (CCL171, Dainippon Pharmaceutical) were suspended in a DME medium (Bio Whittaker) containing 10% fetal calf serum at a concentration of 1 x 10⁵ cells/ml. 500 µl of the suspension was placed into a 48-well cell culture plate. After culturing at 37°C in the presence of 5% CO₂ for 24 hours, the medium was exchanged for a DME medium containing 1% fetal calf serum. Then, one of samples was added thereto. The plate was incubated for additional 24 hours. The medium was then recovered, and the amount of HGF in the culture medium was measured using Quantikine Human Hepatocyte Growth Factor (HGF) ELISA Kit (Funakoshi). The amount of HGF produced was expressed defining a negative control as 100%. In this case, the amount of HGF produced for the negative control was 7.2 ng/ml. Cyclopentenone (at a final concentration of 20, 40 or 160 µM), GM (at a final concentration of 20, 40, 80 or 160 µM), or dipropionylcyclopentenone (at a final concentration of 32 or 64 µM) was added as a sample. As a negative control, distilled water was added in the same volume as the sample. As a positive control, prostaglandin E₁ was added at a final concentration of 0.01, 0.1, 1 or 10 µM.

As a result, the amount of HGF produced by cells to which cyclopentenone or a derivative thereof was added was significantly increased in each case as compared with the negative control to which distilled water was added. In addition, the amount of HGF produced was noticeably increased as compared with the positive control to which prostaglandin E₁ was added. Thus, it was revealed that cyclopentenone and derivatives thereof have stronger activities of enhancing HGF production than that of prostaglandin, which has been confirmed to enhance HGF. The results obtained when cyclopentenone, GM and dipropionylcyclopentenone were added are shown in Tables 5, 6 and 7, respectively.

**Table 5**

| Cyclopentenone concentration (µM) | Increase in HGF production (%) |
|---|---|
| 0 | 100 |
| 20 | 144 |
| 40 | 218 |
| 160 | 238 |

**Table 6**

| GM concentration (µM) | Increase in HGF production (%) |
|---|---|
| 0 | 100 |
| 20 | 154 |
| 40 | 179 |
| 80 | 243 |
| 160 | 319 |

**Table 7**

| Dipropionylcyclopentenone concentration (µM) | Increase in HGF production (%) |
|---|---|
| 0 | 100 |
| 32 | 120 |
| 64 | 144 |

### Example 3

(1) Human newborn foreskin epithelial Hs68 cells (ATCC CRL-1635) were cultured in a D-MEM medium (Bio Whittaker) containing 10% fetal calf serum (FBS; Gibco BRL) in the presence of 5% CO₂ at 37°C to saturation in a culture vessel. Then, the cells were suspended in the medium at a concentration of 3 x 10⁵/ml using a trypsin-EDTA solution (Bio Whittaker). 200 µl of the suspension was dispensed into each well of a 96-well microtiter plate. When the cells were almost saturated in the culture vessel after culturing for 5 days, the medium was discarded and a medium containing 4HCP at a concentration of 40, 100 or 200 µM was added thereto. The culture supernatant was recovered at intervals of 24 hours for up to 96 hours. The effect of 4HCP on enhancement of human insulin-like growth factor-1 (h-IGF-1) production in Hs68 cells was determined using ELISA-Kit for h-IGF-1 (Diagnostic System Labo). The results are shown in Table 8.

**Table 8**

| 4HCP concentration (µM) | Cultivation time (hour) | | | |
|---|---|---|---|---|
| | 24 | 48 | 72 | 96 |
| | Activity of enhancing h-IGF-1 production (ng/ml) | | | |
| 0 | 0 | 0 | 0 | 0 |
| 40 | 0 | 0 | 0 | 0 |
| 100 | 39.9 | 10.0 | 9.6 | 4.4 |
| 200 | 37.9 | 10.4 | 9.9 | 4.2 |

As shown in Table 8, when 4HCP was added at a concentration of 100 µM or more, the activity of enhancing h-IGF production in Hs68 cells became maximal at 24 hours and decreased thereafter as the time went by.
(2) Hs68 cells were cultured in a D-MEM medium containing 10% fetal calf serum in the presence of 5% CO₂ at 37°C to saturation in a culture vessel. Then, the cells were suspended in the medium at a concentration of 3 x 10⁵/ml using a trypsin-EDTA solution. 200 µl of the suspension was dispensed into each well of a 96-well microtiter plate. When the cells were almost saturated in the culture vessel after culturing for 5 to 7 days, the medium was discarded and the medium containing cyclopentenone or GM at a concentration of 0, 2.5, 5, 10 or 20 µM was added thereto.
The culture supernatant was recovered at 1, 3, 6, 12, 24 or 48 hours. The effect of cyclopentenone or GM on the production (enhancement of expression) of h-IGF-1 in Hs68 cells was determined using ELISA-Kit for h-IGF-1.
As a result, when GM was added at a concentration of 10 to 20 µM, production of h-IGF in Hs68 cells was observed 3 hours after the addition. The amount of h-IGF-1 produced 6 hours after the addition was about twice the amount for the control with no addition.
Furthermore, when cyclopentenone was added at a concentration of 10 to 20 µM, h-IGF-1 production was observed from 1-hour to 6 hours after the addition.
(3) Hs68 cells were cultured in a D-MEM medium containing 10% fetal calf serum in the presence of 5% CO₂ at 37°C to saturation in a culture vessel. Then, the cells were suspended in the medium at a concentration of 3 x 10⁵/ml using a trypsin-EDTA solution. 200 µl of the suspension was dispensed into each well of a 96-well microtiter plate. When the cells were almost saturated in the culture vessel after culturing for 5 to 7 days, the medium was discarded and the medium containing 4HCP, a compound of formula (XVIII) (4HCP-GSH) or glutathione (GSH) at a concentration of 0, 25, 50, 100 or 200 µM was added thereto. The culture supernatant was recovered at 1, 3, 6, 12, 24 or 48 hours. The effect on the production (enhancement of expression) of h-IGF-1 in Hs68 cells was determined using ELISA-Kit for h-IGF-1.
As a result, when 4HCP was added at a concentration of 25 to 200 µM, h-IGF production in Hs68 cells became maximal at 1 hour and decreased thereafter as the time went by. The expression of h-IGF-1 was observed for cells treated with 4HCP at a concentration of 100 µM or more at 48 hours. The compound of formula (XVIII) at a concentration ranging from 100 to 200 µM was confirmed to exhibit an activity of enhancing h-IGF-1 in a concentration-dependent manner. The results are shown in Tables 9 and 10. Significant enhancement of h-IGF-1 production was observed when cultured for 10 to 30 minutes. On the other hand, no significant activity of enhancing h-IGF-1 was observed for the medium to which glutathione alone was added.

**Table 9:**

| Activity of enhancing h-IGF-1 production by 4HCP (ng/ml) | | | | | | |
|---|---|---|---|---|---|---|
| Amount of 4HCP added (µM) | Cultivation time (hour) | | | | | |
| | 1 | 3 | 6 | 12 | 24 | 48 |
| | Activity of enhancing IGF-1 production (ng/ml) | | | | | |
| 0 | 7.2 | 8.9 | 6.3 | 0 | 0 | 0 |
| 25 | 30.6 | 29.5 | 9.6 | 0 | 0 | 0 |
| 50 | 49.5 | 42.1 | 27.8 | 0 | 0 | 0 |
| 100 | 52.8 | 47.2 | 31 | 22.1 | 25.6 | 32 |
| 200 | 59.2 | 51.7 | 44.4 | 42.1 | 50.3 | 50.2 |

**Table 10:**

| Activity of enhancing h-IGF-1 production by 4HCP-GSH (ng/ml) | | | | | | |
|---|---|---|---|---|---|---|
| Amount of 4HCP-GSH added (µM) | Cultivation time (hour) | | | | | |
| | 1 | 3 | 6 | 12 | 24 | 48 |
| | Activity of enhancing IGF-1 production (ng/ml) | | | | | |
| 0 | 7.2 | 8.9 | 6.3 | 0 | 0 | 0 |
| 100 | 8.4 | 12 | 10.6 | 6.1 | 6.7 | 1.4 |
| 200 | 33.3 | 37.9 | 24.7 | 20.4 | 20.9 | 27.8 |

### Example 4

A model of atrophy in hind limb muscle was prepared by crushing sciatic nerves of a male Wistar rat of 5 weeks old. Weight of astrocnemius muscle of hind limb removed 2 weeks after the crushing was measured, and was expressed as a ratio of the weight of the muscle of crushed limb to that of the normal limb.

A 10% (w/v) aqueous solution of DNA•Na (Nichiro) of which the pH had been adjusted to 5 by adding diluted HCl was heated at 120°C using an autoclave for 3 hours to prepare a heat treatment product of DNA.

A 10-fold dilution (about 11 mg/kg/day of 4HCP) and a 100-fold dilution (about 1mg/kg/day of 4HCP) of the heat treatment product of DNA in water were prepared, and given to the rats as drinking water starting from 3 days before the crushing (N=9-10). Tap water was given to a control group (N=10).

The results are shown in Table 11. The numbers in Table 11 represent mean ± standard deviation. The mark * in the table means significant difference with significance level of 5% or less as compared with the control group.

In the control group, prominent muscular atrophy was caused by the crushing, and almost no recovery was observed even at 2 weeks after the crushing. On the other hand, recovery of muscle weight was promoted in a dose-dependent manner for the group to which the heat treatment product of DNA was given as drinking water. The muscular atrophy state observed for the control group was ameliorated for the group to which the 10-fold dilution of the heat treatment product of DNA was given as drinking water due to the significant effect of increasing/strengthening muscle.

**Table 11**

| | Muscle weight (%) Mean ± standard deviation |
|---|---|
| Control (N=10) | 49.0 ± 2.58 |
| 100-fold dilution of heat | 49.8 ± 1.16 |
| treatment product of DNA (N=9) | |
| 10-fold dilution of heat | 58.2 ± 2.64* |
| treatment product of DNA (N=10) | |

| | |
|---|---|
| *;p<0.05 vs. control group | |

### Example 5

(1) Injectable preparation
   Cyclopentenone or GM was added to saline at a concentration of 1% to prepare an injectable preparation.
(2) Tablet
   Tablets each containing 100 mg of dipropionylcyclopentenone or 4HCP and a suitable amount of crystallite cellulose were prepared, and coated with sugar.

### Referential Example 6

ddY mouse (female, 5 weeks old, weighing about 25 g) purchased from Japan SLC was used for experiments after preliminary breeding for 1 week. 1 x 10⁶ Ehrlich cancer cells were intraperitoneally inoculated into the mouse. 10 mg/kg of cyclopentenone was intraperitoneally administered 1 day after the inoculation. The mouse was sacrificed by decapitating and exsanguinating 8 or 12 days after the inoculation with cancer cells. 2 ml, which is the same volume as that of ascites retained in the control mouse, of PBS (-) (Nissui Pharmaceutical) containing 1% bovine serum albumin (Sigma) was intraperitoneally injected into the mouse, which was recovered after extensive massage. For a control group, ascites was directly recovered. The recovered ascites and peritoneal lavage fluid were centrifuged at 2000 rpm for 5 minutes to recover supernatants. The supernatants were used to quantify the amounts of interleukin-12 using an ELISA kit for mouse interleukin-12 measurement (Endogen).

The amounts of intraperitoneal interleukin-12 for the respective mice at 8 days and 12 days after the inoculation with cancer cells are shown in Table 12. The amount was below detection limit for all of the 3 mice in the control group at 8 days after the inoculation with cancer cells. On the other hand, an activity of enhancing interleukin-12 production by cyclopentenone was observed for each mouse in the group administered with cyclopentenone, indicating that T cells and NK/LAK cells in local sites were activated. Furthermore, interleukin was still observed in a peritoneal cavity for the group administered with cyclopentenone at 12 days after the inoculation with cancer cells, indicating that an antitumor effect due to immunostimulation was exhibited. Cyclopentenone derivatives also exhibited activities of enhancing interleukin-12 production as well.

**Table 12**

| | Amount of intraperitoneal interleukin-12 (pg/ml) | | | | | |
|---|---|---|---|---|---|---|
| | Control group | | | Group administered with cyclopentenone | | |
| After 8 Days | ND | ND | ND | 11.0 | 100.0 | 25.0 |
| After 12 Days | | All dead | | 43.0 | 97.2 | 66.4 |
| ND: Not detected. | | | | | | |

### Industrial Applicability

The present invention provides a pharmaceutical composition- containing, as an active ingredient, a compound that exhibits an activity of enhancing growth factor production and/or interleukin-12 production, said pharmaceutical composition being effective for a disease that requires enhancement of growth factor production for its treatment or prevention and/or a disease that requires enhancement of interleukin-12 production for its treatment or prevention. The pharmaceutical composition has an activity of enhancing HGF production, NGF production, h-IGF production, interleukin-12 production or the like in vivo. Thus, the pharmaceutical composition is useful as a pharmaceutical composition for treating or preventing diseases that require enhancement of production of such growth factors for their treatment or prevention and/or diseases that require enhancement of production of interleukin-12 for their treatment or prevention, including hepatitis, Alzheimer's disease, diabetes and cancer.

Furthermore, it is now possible to produce a food or a drink using a substance having an ability to enhance growth factor production and/or interleukin-12 production, said substance being selected from the group consisting of materials containing cyclopentenone, materials containing cyclopentenone derivatives, materials containing 4HCP and materials containing 4HCP derivatives. Thus, when the food or drink is taken on a routine basis, the disease state of a disease that requires enhancement of growth factor production for its treatment or prevention and/or a disease that requires enhancement of interleukin-12 production for its treatment or prevention can be ameliorated.

Accordingly, a functional food or drink containing, as an active ingredient, a substance selected from the group consisting of materials containing cyclopentenone, materials containing cyclopentenone derivatives, materials containing 4HCP and materials containing 4HCP derivatives is useful for maintaining homeostasis of a living body based on its activity of enhancing growth factor production and/or interleukin-12 production.

The present invention also provides a composition for enhancing growth factor production and/or interleukin-12 production. The composition is useful for studying functions of growth factors, and for screening medicines for diseases related to growth factors.

Furthermore, the present invention provides a composition for increasing/strengthening muscle. The composition is useful for treatment of diseases due to neurothlipsis.

## Claims

1. A pharmaceutical composition for treating or preventing a disease that requires enhancement of growth factor production for its treatment or prevention and/or a disease that requires enhancement of interleukin-12 production for its treatment or prevention, the pharmaceutical composition containing, as an active ingredient, a compound selected from the group consisting of 4,5-dihydroxy-2-cyclopenten-1-one of formula (I): 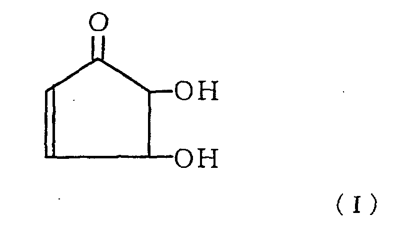 , 4-hydroxy-2-cyclopenten-1-one and derivatives thereof.

2. A composition for enhancing growth factor production and/or interlukin-12 production containing, as an active ingredient, a compound selected from the group consisting of 4,5-dihydroxy-2-cyclopenten-1-one of formula (I), 4-hydroxy-2-cyclopenten-1-one and derivatives thereof.

3. A food or a drink for enhancing growth factor production and/or interluekin-12 production which contains, which is produced by diluting and/or which is produced by adding thereto a substance selected from the group consisting of materials containing 4,5-dihydroxy-2-cyclopenten-1-one of formula (I), materials containing 4-hydroxy-2-cyclopenten-1-one, materials containing 4,5-dihydroxy-2-cyclopenten-1-one derivatives and materials containing 4-hydroxy-2-cyclopenten-1-one derivatives.
